# EUROPEAN PATENT APPLICATION

(11) **EP 3 546 597 A1**
(43) Date of publication of application: **02.10.2019**
(21) Application number: 18164516.9
(22) Date of filing: 28.03.2018
(51) Int. Cl.: C12Q 1/6876, C12Q 1/6883

(54) **BIOMARKER PANEL FOR NAFLD/NASH**

(71) Applicant: Sanofi, 75008 Paris (FR); Centre Hospitalier Régional et Universitaire de Lille, 59000 Lille (FR); Université de Lille, 59800 Lille (FR); Institut National de la Santé et de la Recherche Médicale, 75013 Paris (FR)
(72) Inventor: The designation of the inventor has not yet been filed
(74) Representative: Altmann Stößel Dick Patentanwälte PartG mbB

(57) **Abstract**

The present invention relates generally to the field of molecular biology and concerns a method for monitoring a treatment of non-alcoholic steatohepatitis (NASH) in a subject suffering from NASH. The method is based on the determination of the amounts of at least three biomarkers selected from the group consisting of miR-193b, miR-365a, miR-99a, miR-28, let-7c, miR-192, miR-215, and miR-122 in a first and second sample from said subject. The present invention further concerns a method for diagnosing non-alcoholic steatohepatitis (NASH) in a subject suspected to suffer from NASH and method for assessing whether a test compound is capable of treating NASH.

SEQUENCE LISTING

<110> SANOFI
      Centre Hospitalier Régional et Universitaire de Lille
      Université de Lille
      Institut National de la Santé et de la Recherche Médicale

<120> Biomarker panel for NAFLD/NASH

<130> SAN14762EP

<160> 11

<170> BiSSAP 1.3.6

<210> 1
<211> 22
<212> RNA
<213> Homo sapiens

<220>
<223> miR-193b

<400> 1
aacuggcccu caaagucccg cu                                              22

<210> 2
<211> 22
<212> RNA
<213> Homo sapiens

<220>
<223> miR-365a

<400> 2
uaaugccccu aaaaauccuu au                                              22

<210> 3
<211> 22
<212> RNA
<213> Homo sapiens

<220>
<223> miR-99a

<400> 3
aacccguaga uccgaucuug ug                                              22

<210> 4
<211> 22
<212> RNA
<213> Homo sapiens

<220>
<223> miR-28

<400> 4
cacuagauug ugagcuccug ga                                              22

<210> 5
<211> 22
<212> RNA
<213> Homo sapiens

<220>
<223> let-7c

<400> 5
ugagguagua gguuguaugg uu                                              22

<210> 6
<211> 21
<212> RNA
<213> Homo sapiens

<220>
<223> miR-192

<400> 6
cugaccuaug aauugacagc c                                               21

<210> 7
<211> 21
<212> RNA
<213> Homo sapiens

<220>
<223> miR-215

<400> 7
augaccuaug aauugacaga c                                               21

<210> 8
<211> 22
<212> RNA
<213> Homo sapiens

<220>
<223> miR-122

<400> 8
uggaguguga caaugguguu ug                                              22

<210> 9
<211> 18
<212> RNA
<213> Homo sapiens

<220>

<223> miR-1260a

<400> 9
aucccaccuc ugccacca                                                    18

<210> 10
<211> 22
<212> RNA
<213> Homo sapiens

<220>
<223> miR-30a

<400> 10
uguaaacauc cucgacugga ag                                               22

<210> 11
<211> 22
<212> RNA
<213> Homo sapiens

<220>
<223> miR-34a

<400> 11
caaucagcaa guauacugcc cu                                               22

## Description

### FIELD OF THE INVENTION

The present invention relates generally to the field of molecular biology and concerns a method for monitoring a treatment of non-alcoholic steatohepatitis (NASH) in a subject suffering from NASH. The method is based on the determination of the amounts of at least three biomarkers selected from the group consisting of miR-193b, miR-365a, miR-99a, miR-28, let-7c, miR-192, miR-215, and miR-122 in a first and second sample from said subject. The present invention further concerns a method for diagnosing non-alcoholic steatohepatitis (NASH) in a subject suspected to suffer from NASH and method for assessing whether a test compound is capable of treating NASH. Further encompassed by the present invention are compositions and kits which comprise detection agents for at least three of the aforementioned biomarkers.

### BACKGROUND OF THE INVENTION

Nonalcoholic steatohepatitis (NASH) is a significant form of chronic liver disease in adults and children. The natural history of NASH ranges from indolent to end-stage liver disease. Current studies are focusing on identification of histologic and/or clinical markers of progression. NASH may be an underlying cause of cryptogenic cirrhosis, and the lesions of NASH may recur in allograft livers. Various clinical conditions and pathogenetic mechanisms have been identified, but many cases remain idiopathic; lack of significant alcohol use is, by definition, common to all cases. Neither clinical evaluation nor laboratory values can ensure either the diagnosis or the exclusion of NASH, and liver biopsy interpretation continues to be considered the "gold standard" for diagnosis. The lesions in NASH are similar but not identical to those of alcoholic steatohepatitis; exact, specific histologic criteria for the diagnosis are currently under discussion. The lesions most commonly accepted for NASH include steatosis, hepatocyte ballooning degeneration, mild diffuse lobular mixed acute and chronic inflammation, and perivenular, perisinusoidal collagen deposition.

Liver biopsy such as percutaneous liver biopsy has remained the criterion standard or "gold standard" in the evaluation of NASH. Other biopsy methods are typically even more invasive and include transvenous and laparoscopic liver biopsy (Am J Gastroenterol. 94: 2467-74, Brunt 2010, Nat Rev Gastroenterol Hepatol. 7:195-203). Although liver biopsies are generally regarded as safe, they bare risks that are potentially lethal. Approximately 2% of patients undergoing liver biopsy require hospitalization for the management of an adverse event. Significant bleeding after a liver biopsy occurs in approximately 1% of patients who are biopsied. If bleeding persists, a blood transfusion may be needed. Surgery or angiography, where the bleeding site is identified and treated, may be required if the bleeding is severe or does not stop on its own. Intraperitoneal haemorrhage is the most serious consequence of bleeding. Further, liver biopsies are associated with high costs.

Currently, there are no commercially available non-invasive assays for the assessment of NASH which are based on the determination of biomarkers. Clinical liver parameters such as AST, ALT and GGT do not allow for a differentiation between patients suffering from NASH and patients not suffering from NASH.

miRNAs are small, non-coding RNAs (∼18-25 nucleotides in length) that regulate gene expression on a post-transcriptional level by degrading mRNA molecules or blocking their translation. They are stable in plasma/serum, where they circulate mostly outside of exo-somes, and have been reported as differentially expressed in plasma or serum in different diseases and disorders.

For example, various studies describe the analysis of miRNA expression levels in patients with liver disease.

Becker discloses the evaluation of serum profiles of four microRNAs in two cohorts consisting of 137 NAFLD patients and 61 healthy controls (miR-122, miR-192, miR-21, miR-223). Correlation of microRNA appearance with known biomarkers like ALT and CK18-Asp396 was evaluated. A scoring model was developed, combining the levels of microRNA in circulation and CK18-Asp396 fragments (Becker et al. (2015) PLoS ONE 10(11): e0142661).

Pirzola discloses the global profiling of serum miRNAs with a human serum and plasma miRNA PCR-based array in serum samples from healthy donors and patients with NAFLD. Among 84 circulating miRNAs, miR-122, miR-192, miR-19a and miR-19b, miR-125b, and miR-375 were upregulated >2-fold either in simple steatosis (SS) or non-alcoholic steatohepatitis (NASH). Liver miR-122 expression was 10-fold downregulated in NASH compared with SS (Pirola et al. (2015). Gut, 64(5), 800-812).

WO 2016/196945 discloses a method for the diagnosis of NASH based on the determination of a panel of miRNAs.

WO 2017/046181 discloses a method for the diagnosis of non-alcoholic steatohepatitis (NASH) or for determining the activity, the stage, or the severity of NASH in a subject. The method is based on the determination of biomarkers such as alpha 2 macroglobulin, glycated haemoglobin (HbAlc), fasting glucose, fructosamine, N-terminal pro-peptide of collagen type III, hsa-miR-34a-5p, hsa-miR-122, hsa-miR-192-5p and/or hsa-miR-200a-3p.

According to Trevesani, microRNA 193b-3p is a predictive biomarker of chronic kidney disease (British Journal of Cancer (2016) 115, 1343-1350).

Belarbi discloses that three miRNAs (miR-193b/-126/-26a) cause increased adiponectin secretion when overexpressed in human adipocytes (Belarbi et al., J Clin Endocrinol Metab. 2015, 100(8):E1084-8. doi: 10.1210/jc.2015-1530).

Párrizas discloses that circulating miR-192 and miR-193b are markers of prediabetes and are modulated by an exercise intervention (Párrizas et al., J Clin Endocrinol Metab. 2015 Mar;100(3):E407-15. doi: 10.1210/jc.2014-2574).

Xu discloses that miR-193b was significantly down-regulated in HCC (hepatocellular carcinoma cells) tissue compared to the matching non-tumoral liver tissue (Xu et al., Eur J Cancer. 2010, 46(15):2828-36. doi: 10.1016/j.ejca.2010.06.127).

An effective monitoring of the treatment of NASH based on a minimal invasive test is currently not possible. Further, a robust minimal invasive test for reliably and efficiently diagnosing NASH is needed. In particular, there is a need to identify new marker panels which allow for an improved assessment of NASH.

Accordingly, the technical problem underlying the present invention could be seen as the provision of means and methods for monitoring a treatment of NASH and for diagnosing NASH. The technical problem is solved by the embodiments characterized in the claims and herein below.

### BRIEF SUMMARY OF THE PRESENT INVENTION

The present invention relates to a method for monitoring a treatment of non-alcoholic steatohepatitis (NASH) in a subject suffering from NASH, comprising
determining the amounts of at least three biomarkers selected from the group consisting of miR-193b, miR-365a, miR-99a, miR-28, let-7c, miR-192, miR-215, and miR-122 in a first sample which has been obtained from said subject before or after initiating said treatment, determining the amounts of said at least three biomarkers in a second sample which has been obtained from said subject after said first sample, and
comparing the amounts of said at least three biomarkers in said second sample to the amounts of said at least three biomarkers in said first sample, thereby monitoring the treatment of NASH.

The present invention further concerns a method for diagnosing non-alcoholic steatohepatitis (NASH) in a subject suspected to suffer from NASH, comprising
determining the amounts of at least three biomarkers selected from the group consisting of miR-193b, miR-365a, miR-99a, miR-28, let-7c, miR-192, miR-215, and miR-122 in a sample from said subject, and
comparing the amounts of said at least three biomarkers to a reference, thereby diagnosing NASH.

Preferably, the comparison of the amounts of said at least three biomarkers to a reference in step b) comprises the comparison of a score calculated based on the amounts of said at least three biomarkers to a reference score.

The present invention further relates to a method for assessing whether a test compound is capable of treating NASH, comprising:
determining the amounts of at least three biomarkers selected from the group consisting of miR-193b, miR-365a, miR-99a, miR-28, let-7c, miR-192, miR-215, and miR-122 in a first sample which has been obtained from a subject suffering from NASH before or after initiating administration of said test compound,
determining the amounts of said at least three biomarkers in a second sample which has been obtained from said subject after said first sample, and
comparing the amounts of said at least three biomarkers in said second sample to the amount of said at least three biomarkers in said first sample, thereby assessing whether said test compound is capable of treating NASH.

The present invention also deals with the *in vitro* use of at least three biomarkers selected from the group consisting of miR-193b, miR-365a, miR-99a, miR-28, let-7c, miR-192, miR-215, and miR-122 as biomarkers, or of at least three detection agents selected from the group consisting of a detection agent which specifically detects miR-193b, a detection agent which specifically detects miR-365a, a detection agent which specifically detects miR-99a, a detection agent which specifically detects miR-28, a detection agent which specifically detects let-7c, a detection agent which specifically detects miR-192, a detection agent which specifically detects miR-215, and a detection agent which specifically detects miR-122,
for monitoring a treatment of NASH in a subject suffering from NASH,
for diagnosing whether a subject who is suspected to suffer from NASH suffers from NASH, or
for assessing whether a test compound is capable of treating NASH.

In an embodiment of the methods and uses of the present invention, the subject is human.

In an embodiment of the methods or uses of the present invention, the sample is a blood, serum or plasma sample.

In an embodiment of the present invention, one of the at least three biomarkers is miR-193b. In another preferred embodiment, at least the amounts of the biomarkers miR-193b and miR-365a are determined. In a particularly preferred embodiment, the amounts of biomarkers miR-193b, miR-365a and miR-99a are determined.

In an embodiment of the methods or uses of the present invention, the methods and uses further comprise the determination of the amount of the cytokeratin 18 fragment (M30) in the sample(s).

In an embodiment of the methods or uses of the present invention, the methods and uses according to the present invention further comprise the determination of the amount of at least one further miRNA biomarker selected from the group consisting of miR-1260a, miR-30a, and miR-34a in the sample(s).

The present invention further concerns a composition comprising at least three detection agents selected from the group consisting of a detection agent which specifically detects miR-193b, a detection agent which specifically detects miR-365a, a detection agent which specifically detects miR-99a, a detection agent which specifically detects miR-28, a detection agent which specifically detects let-7c, a detection agent which specifically detects miR-192, a detection agent which specifically detects miR-215, and a detection agent which specifically detects miR-122.

Finally, the present invention relates to a kit comprising at least three detection agents selected from the group consisting of a detection agent which specifically detects miR-193b, a detection agent which specifically detects miR-365a, a detection agent which specifically detects miR-99a, a detection agent which specifically detects miR-28, a detection agent which specifically detects let-7c, a detection agent which specifically detects miR-192, a detection agent which specifically detects miR-215, and a detection agent which specifically detects miR-122.

### DETAILED SUMMARY OF THE PRESENT INVENTION - DEFINITIONS

As set forth above, the present invention concerns a method for monitoring a treatment of non-alcoholic steatohepatitis (NASH) in a subject suffering from NASH, a method for diagnosing non-alcoholic steatohepatitis in a subject suspected to suffer from NASH, and a method for assessing whether a test compound is capable of treating NASH.

The definitions and explanations provided in connection with the method of monitoring a treatment of NASH preferably apply *mutatis mutandis* to the method for diagnosing non-alcoholic steatohepatitis, and the method for assessing whether a test compound is capable of treating NASH and *vice versa,* except if stated otherwise.

The methods of the present invention are preferably carried out *in vitro,* i.e. are not practiced on the human or animal body. Moreover, they may comprise steps in addition to those explicitly mentioned above. Further steps may relate to sample pre-treatment or evaluation of the diagnostic results obtained by the methods. Preferred further evaluation steps are described elsewhere herein. The methods may partially or entirely be assisted by automation. For example, steps pertaining to the determination of the amount of a biomarker can be automated by robotic and automated reader devices. Likewise, steps pertaining to a comparison of amounts can be automated by suitable data processing devices, such as a computer, comprising a program code which when being executed carries out the comparison automatically. A reference in such a case will be provided from a stored reference, e.g., from a database.

Non-alcoholic steatohepatitis (NASH) is the most extreme form of NAFLD (Non-alcoholic fatty liver disease). NAFLD is one of the types of fatty liver which occurs when fat is deposited in the liver due to causes other than consumption of alcohol. The deposition of fat is also referred to as steatosis. Patients suffering from NASH frequently have obesity, type 2 diabetes mellitus, dyslipidemia, and/or the metabolic syndrome. Symptoms of NASH are fatigue, malaise, or right upper quadrant abdominal discomfort. Hepatomegaly develops in a larger number of patients. Splenomegaly may develop if advanced hepatic fibrosis is present. Most NASH patients are, however, asymptomatic. Preferably, a subject suffers from NASH, it the fibrosis score is equal to or larger than 3 and/or if the ballooning score is equal to or larger than 1. The fibrosis score and the ballooning score are well-known in the art and can be determined b the skilled person without further ado. E.g., the fibrosis score and the ballooning score are determined as described in Kleiner et al. for the Nonalcoholic Steatohepatitis Clinical Research Network. Design and validation of a histological scoring system for nonalcoholic fatty liver disease. Hepatology 41:1313-1321, 2005.

In an embodiment of the present invention, the test subject does not suffer from liver cancer.

The present invention inter alia relates to a method for monitoring a treatment of non-alcoholic steatohepatitis (NASH). Treatments of NASH are well-known in the art.

In a preferred embodiment, the treatment of NASH is gastric bypass surgery (frequently also referred to as bariatric surgery), see de Almeida et al. OBES SURG (2006) 16: 270, or Hafeeez, Journal of Obesity, Volume 2013 (2013), Article ID 839275.

In another preferred embodiment, said treatment of NASH is a drug-based treatment. Preferably, said drug-based treatment comprises the administration of at least one drug selected from statins, incretin analogues, thiazolidinediones, and orlistat.

The expression "monitoring a treatment of NASH" as used herein refers to assessing whether the subject responds to the treatment of NASH, or not. Accordingly, the efficacy of a treatment is assessed. Preferably, a subject responds to said treatment, if the NASH status of the subject has improved (in the period between the first and the second sample). Preferably, a subject does not respond to said treatment, if the NASH status of the subject has not improved (in the period between the first and the second sample). Whether the NASH status has improved, or not, can be determined by assessing liver histology parameters and clinical liver parameters as described in the Examples section.

By monitoring a treatment of NASH, decisions can be made whether the treatment shall be continued, stopped or amended. Thus, the method may comprise the further step of continuing, stopping or amending said treatment.

The term "diagnosing" as used herein refers to assessing whether a subject suffers from NASH, or not. In particular, the term refers to assessing the probability according to which a subject is suffering from NASH, or not.

The expression "assessing whether a test compound is capable of treating NASH" as used herein refers to determining whether a test compound can be applied for the treatment of NASH, or not. By carrying out this assessment, a drug for the treatment of NASH can be identified. The method may assist clinical trials.

As will be understood by those skilled in the art, the assessments as referred to herein, i.e. the monitoring, the diagnosis and the assessment whether a test compound is capable of treating NASH, are usually not intended to be correct for 100% of the subjects or test compounds to be assessed. Preferably, the term, however, requires that a statistically significant portion of subjects (e.g., a cohort in a cohort study) can be monitored or diagnosed. Whether a portion is statistically significant can be determined without further ado by the person skilled in the art using various well-known statistic evaluation tools, e.g., determination of confidence intervals, p-value determination, Student's t-test, Mann-Whitney test etc.. Preferred confidence intervals are at least 90%, at least 95%, at least 97%, at least 98%, or at least 99%. The p-values are, preferably, 0.1, 0.05, 0.01, 0.005, or 0.0001.

As set forth above, the assessments as referred to herein may comprise further steps such as the confirmation of the monitoring, the confirmation of a diagnosis, or the confirmation that a test compound is capable of treating NASH. Accordingly, the terms "diagnosing" and "monitoring" in the context of the present invention, in particular, encompass aiding a physician in the diagnosis of NASH or in the monitoring of NASH. Thus, the methods of the present invention can be considered as an adjunct to other clinical diagnostic evaluations. The skilled person is aware that the results obtained by carrying out the methods of the present invention are preferably interpreted in conjunction with further clinical information on the condition of the subject in order to establish a final diagnosis/assessment.

The "subject" as referred to herein is, preferably, a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In a preferred embodiment, the subject is a human subject.

In a preferred embodiment of the method of monitoring a treatment of NASH and the method of assessing whether a test compound is capable of treating NASH, the sample is from a subject who suffers from NASH. In a preferred embodiment of the method of diagnosing NASH, the sample is from a subject who is suspected to suffer from NASH. Preferably, a subject is suspected to suffer from NASH is a subject who shows some or all of the symptoms associated with NASH. Also preferably, a subject is suspected to suffer from NASH if the subject has at least one risk factor for NASH, in particular obesity, type 2 diabetes mellitus, or dyslipidemia, and/or if the subject has an elevated aminotransferase level.

Thus, it is in particular envisaged that the subject to be tested thus suffers from diabetes mellitus. The term "diabetes mellitus", preferably, refers to type 2 diabetes mellitus. The term is well-known in the art. In type 2 diabetes insulin secretion is inadequate. Often insulin levels are very high, especially early in the disease, but peripheral insulin resistance and increased hepatic production of glucose makes insulin levels inadequate to normalized plasma glucose levels. Insulin production then falls, further exacerbating hyperglycemia.

Further, it is envisaged that the subject is obese. Accordingly, the test subject has preferably a body mass index of larger than 30 kg/m². More preferably, the test subject has a body mass index of larger than 34.8 kg/m², even more preferably, of larger than 40 kg/m², and most preferably, of larger than 45 kg/m².

The term "sample" refers to a sample of a body fluid, to a sample of separated cells or to a sample from a tissue or an organ. Samples of body fluids can be obtained by well-known techniques and include samples of blood, plasma, serum, urine, lymphatic fluid, sputum, ascites, or any other bodily secretion or derivative thereof. Tissue or organ samples may be obtained from any tissue or organ by, e.g., biopsy. Separated cells may be obtained from the body fluids or the tissues or organs by separating techniques such as centrifugation or cell sorting. E.g., cell-, tissue- or organ samples may be obtained from those cells, tissues or organs which express or produce the biomarker. The sample may be frozen, fresh, fixed (e.g. formalin-fixed), centrifuged, and/or embedded (e.g. paraffin-embedded), etc. The cell sample can, of course, be subjected to a variety of well-known post-collection preparative and storage techniques (e.g., nucleic acid and/or protein extraction, fixation, storage, freezing, ultrafiltration, concentration, evaporation, centrifugation, etc.) prior to assessing the amount of the biomarker(s) in the sample.

In a preferred embodiment of the present invention, the sample is a blood (i.e. whole blood), serum or plasma sample. In particular, the sample is a serum sample.

Serum is the liquid fraction of whole blood that is obtained after the blood is allowed to clot. For obtaining the serum, the clot is removed by centrifugation and the supernatant is collected. Plasma is the acellular fluid portion of blood. For obtaining a plasma sample, whole blood is collected in anticoagulant-treated tubes (e.g. citrate-treated or EDTA-treated tubes). Cells are removed from the sample by centrifugation and the supernatant (i.e. the plasma sample) is obtained. If a plasma sample is used, the sample is preferably subjected to a centrifugation step in order to remove potential contaminations.

In a preferred embodiment of the present invention, the sample is a fasting sample, in particular a fasting blood, serum or plasma sample. Accordingly, the sample shall have been obtained from a fasting subject. A fasting subject, in particular, is a subject who refrained from food and beverages, except for water, prior to obtaining the sample to be tested. Preferably, a fasting subject refrained from food and beverages, except for water, for at least eight hours prior to obtaining the sample to be tested. More preferably, the sample has been obtained from the subject after an overnight fast.

The aforementioned samples are, preferably, pre-treated before they are used for the method of the present invention. In a preferred embodiment of the method of the present invention, the sample is further processed by well-known methods in order to further enrich and/or purify miRNA molecules that are present in the sample. Preferably, small RNAs, e.g. RNAs being about 18 to 200 nt in length are enriched. For enriching small RNAs, serum or plasma samples are lyzed with a suitable reagent. After addition of chloroform, the lysate can be separated into aqueous and organic phases by centrifugation. RNA partitions to the upper, aqueous phase, while DNA partitions to the interphase and proteins to the lower, organic phase or the interphase. Preferably, the upper aqueous phase is extracted, and ethanol is added to provide appropriate binding conditions for small RNA molecules. The sample could then be transferred to a suitable spin column, where the total RNA binds to the membrane and phenol and other contaminants are efficiently washed away. Small RNAs are eluted with water.

In accordance with the method of monitoring a treatment of NASH and the method of assessing whether a test compound is capable of treating NASH, the amounts of at least three miRNA biomarkers as referred to herein are determined in a first sample and a second sample. The first sample, preferably, has been obtained from said subject before or after initiating said treatment (or before or after initiating administration of said test compound). The second sample preferably has been obtained from said subject after said first sample.

Preferably, the first sample in the context of the present invention has obtained before initiating the treatment (or before initiating administration of said test compound). More preferably, the first sample is obtained shortly before initiating said treatment (administration of said test compound). Even more preferably, the sample has been obtained within one week before initiating said treatment (or before initiating administration of said test compound). Most preferably, the sample has been obtained within 24 hours before initiating said treatment (or before initiating administration of said test compound).

Since the aforementioned method of the present invention comprises the assessment of changes of the amounts of biomarkers that are caused by a treatment, the first sample may also be obtained after initiating said treatment (administration of said test compound).

Thus, in a preferred embodiment, the sample has been obtained within 24 hours (or within 7 days) before initiating said treatment, or after initiating the treatment.

Thus, the second sample, preferably, is obtained (i) after the first sample and (ii) after initiating said treatment (or after initiating administration of said test compound). It is particularly contemplated that the second sample is obtained after a reasonable period of time after obtaining the first sample. It is to be understood that the amounts of markers referred herein, do not instantly change (e.g. within 1 minute or 1 hour) after initiating a treatment or after administering a test compound. Therefore, "reasonable" in this context refers to intervals between obtaining the first and second sample which intervals allow the markers to adjust. Therefore, the second sample, preferably, has been obtained at least one month after said first sample, more preferably at least two months after said first sample, and most preferably at least three months after the first sample. Further, it is envisaged that the second sample has been obtained at least six months after the first sample.

In accordance with the present invention, the amounts of at least three miRNA biomarkers shall be determined in a sample or in samples (i.e. a first and a second sample) from a subject. In particular, the amounts of at least three miRNAs selected from the group consisting of miR-193b, miR-365a, miR-99a, miR-28, let-7c, miR-192, miR-215, and miR-122 are determined.

microRNAs (miRNAs) are single-stranded RNA molecules of app. 21-23 nucleotides in length which regulate gene expression. Said miRNAs are frequently also referred to as mature miRNAs. miRNAs are non-coding RNAs and, thus, are not translated into protein. They are derived from a primary transcript (a pri-miRNA) which is processed in a first step into a short stem-loop (hairpin) structure (pre-miRNA) and then into a functional miRNA. Mature miRNA molecules are partially complementary to corresponding target mRNAs and binding leads to translation inhibition or mRNA degradation. Under the standard nomenclature system, names are assigned to experimentally confirmed miRNAs. The prefix "mir" is followed by a dash and a number. The uncapitalized "mir" refers to the pre-miRNA, while a capitalized "miR" refers to the mature form. miRNAs with nearly identical sequences bar one or two nucleotides are annotated with an additional lower case letter. Species of origin is designated with a three-letter prefix, e.g. hsa for *Homo sapiens* (human). Two mature miRNAs originating from opposite arms of the same pre-miRNA are denoted with a -3p or -5p suffix.

The term "at least three miRNAs" as used herein, means three or more than three miRNAs. Accordingly, the amounts of three, four, five, six, seven, all eight miRNAs as set forth above may be determined in a sample or in samples from a sample (and compared to the amount in the first sample or compared to a reference).

In addition to the eight miRNAs referred to above, it is envisaged to determine the amount of at least one further miRNAs biomarker in the sample(s) from the subject. Said at least one further miRNA biomarker is preferably selected from the group consisting of miR-1260a, miR-30a, and miR-34a.

Accordingly, the method for monitoring a therapy of NASH and the method for assessing whether a test compound is capable of treating NASH preferably further comprises the determination of the amount of said at least one further miRNA biomarker in said first and said second sample. In addition, the methods shall comprise in a further step the comparison of the amount of said at least one further miRNA biomarker in said second sample to the amount in said first sample. Based on the comparison of the amounts of said at least three biomarkers as set forth above and said at least one further miRNA biomarker in said second sample to the amount of said at least three biomarkers and of said at least one further miRNA biomarker in said first sample, the treatment of NASH is monitored, or it is assessed whether the test compound is capable of treating NASH.

Accordingly, the method for diagnosing NASH may further comprise the determination of the amount of said least one further miRNA biomarker in the sample from the subject. Preferably, the method comprises in a further step the comparison of the amount of said at least one further miRNA biomarker in said second sample to the amount in said first sample. Based on the comparison of the amounts of said at least three biomarkers and said at least one further miRNA biomarker to the reference(s), it is diagnosed whether the test subject suffers from NASH or not.

The miRNAs as referred to herein, i.e. miR-193b, miR-365a, miR-99a, miR-28, let-7c, miR-192, miR-215, miR-122, miR-1260a, miR-30a, and miR-34a are preferably human miRNAs (indicated by the prefix "hsa"). The sequences are well-known in the art and can be assessed via the microRNA database miRBase (Release 21, see Kozomara A, Griffiths-Jones S.: miRBase: annotating high confidence microRNAs using deep sequencing data. Nucleic Acids Res. 2014 42:D68-D73).

Preferably, the miRNAs as referred to herein can be either the 3p-strand or the 5p-strand. For example, the biomarker miR-193b can be either hsa-miR-193b-3p or hsa-miR-193b-5p. However, it is preferred that miR-193b is hsa-miR-193b-3p, i.e. it is preferred determine the amount of the 3p-strand of human miR-193b.

Table 1 lists the preferred miRNA strand for each of the miRNA biomarkers as referred to herein. Furthermore, the table provides an overview on the nucleic acid sequences of the miRNAs. The sequences for the preferred strands are shown.

**Table 1: Sequences of miRNA biomarkers**

| miRNA | Preferred miRNA | Sequence of the preferred miRNA | Sequence identifier |
|---|---|---|---|
| miR-193b | hsa-miR-193b-3p | AACUGGCCCUCAAAGUCCCGCU | SEQ ID NO: 1 |
| miR-365a | hsa-miR-365a-3p | UAAUGCCCCUAAAAAUCCUUAU | SEQ ID NO: 2 |
| miR-99a | hsa-miR-99a-5p | AACCCGUAGAUCCGAUCUUGUG | SEQ ID NO: 3 |
| miR-28 | hsa-miR-28-3p | CACUAGAUUGUGAGCUCCUGGA | SEQ ID NO: 4 |
| let-7c | hsa-let-7c-5p | UGAGGUAGUAGGUUGUAUGGUU | SEQ ID NO: 5 |
| miR-192 | hsa-miR-192-5p | CUGACCUAUGAAUUGACAGCC | SEQ ID NO: 6 |
| miR-215 | hsa-miR-215-5p | AUGACCUAUGAAUUGACAGAC | SEQ ID NO: 7 |
| miR-122 | hsa-miR-122-5p | UGGAGUGUGACAAUGGUGUUUG | SEQ ID NO: 8 |
| miR-1260a | hsa-miR-1260a | AUCCCACCUCUGCCACCA | SEQ ID NO: 9 |
| miR-30a | hsa-miR-30a-5p | UGUAAACAUCCUCGACUGGAAG | SEQ ID NO: 10 |
| miR-34a | hsa-miR-34a-3p | CAAUCAGCAAGUAUACUGCCCU | SEQ ID NO: 11 |

It follows from the table, that miR-193b-3p has preferably a sequence as shown in SEQ ID NO: 1 (AACUGGCCCUCAAAGUCCCGCU).

The miRNA-biomarkers are listed in Table 1 in decreasing order of preference. Accordingly, the most preferred miRNA to be determined in accordance with the methods of the present invention is preferably miR-193b, followed by miR-365a, followed by miR-99a etc.

Thus, in a preferred embodiment of the method of the present invention, miR-193b is one of the at least three biomarkers. Thus, the amount of this biomarker shall be determined in the sample(s) and compared (to a reference or to the amount in the first sample). In addition, at least two of the remaining markers (miR-365a, miR-99a, miR-28, let-7c, miR-192, miR-215, and miR-122) have to be determined.

In another preferred embodiment of the methods of the present invention, miR-365a is one of the at least three biomarkers to be determined. In addition, at least two of the remaining markers (miR-193b, miR-99a, miR-28, let-7c, miR-192, miR-215, and miR-122) have to be determined.

Of the 681 miRNAs tested in the studies underlying the present invention, miR-193b and miR-365a showed the strongest correlation with all tested clinical liver parameters and all tested histological parameters. Accordingly, these two miRNAs are the most preferred biomarkers. Thus, in a particularly preferred embodiment, the biomarkers miR-193b and miR-365a are two of the at least three biomarkers to be determined. Thus, the amounts of the biomarkers miR-193b and miR-365a are determined. In addition, at least one of the remaining markers (miR-99a, miR-28, let-7c, miR-192, miR-215, and miR-122) has to be determined.

In particular, at least the following three biomarkers are determined.
- miR-193b, miR-365a, and miR-99a
- miR-193b, miR-365a, and miR-28
- miR-193b, miR-365a, and let-7c
- miR-193b, miR-365a, and miR-122

In another preferred embodiment, it is envisaged to determine the amounts of at least miR-193b, miR-122, and miR-99a.

In another preferred embodiment, it is envisaged to determine the amounts of at least miR-365a, miR-122, and miR-99a.

In another preferred embodiment, it is envisaged to determine the amounts of at least miR-193b, miR-99a, and miR-28.

In another preferred embodiment, it is envisaged to determine the amounts of at least miR-193b, miR-99a, and miR-1260a.

In another preferred embodiment, it is envisaged to determine the amounts of at least miR-193b, miR-365a, miR-99a, and miR-122.

In another preferred embodiment, it is envisaged to determine the amounts of at least miR-193b, miR-365a, miR-99a, and miR-28.

In another preferred embodiment, it is envisaged to determine the amounts of at least miR-193b, miR-365a, miR-99a, and miR-1260a.

In another preferred embodiment, it is envisaged to determine the amounts of at least miR-193b, miR-365a, miR-99a, miR-28, and miR-122.

In another preferred embodiment, it is envisaged to determine the amounts of at least miR-193b, miR-365a, miR-99a, miR-1260a, and miR-122.

In a further preferred embodiment, the amounts of miR-193b, miR-365a, miR-99a, miR-28, let-7c, miR-192, miR-215, and miR-122 are determined.

In another preferred embodiment, the amounts of all 11 miRNAs listed in the above table are determined. Accordingly, it is envisaged to determine the amounts of miR-193b, miR-365a, miR-99a, miR-192, miR-122, miR-28, miR-1260a, miR-34a, let-7c, miR-215, and miR-30a. Further, it is envisaged to determine all miRNA biomarker assigned with an "up" in Table A.

The amount of a miRNA can be determined in a sample of a subject by techniques well-known in the art. Depending on the nature of the sample, the amount may be determined by PCR-based techniques for quantifying the amount of a polynucleotide or (next generation) sequencing Cissell KA, Deo SK. Trends in microRNA detection. Anal Bioanal Chem. 2009;394(4):1109-1116 or de Planell-Saguer M, Rodicio MC. Analytical aspects of microRNA in diagnostics: a review. Anal Chim Acta 2011 Aug 12;699(2):134-52). For example, the amounts of a miRNA biomarker can be determined as described in Mitchell (Mitchell PS, Parkin RK, Kroh EM, et al. Circulating microRNAs as stable blood-based markers for cancer detection. Proc Natl Acad Sci U S A 2008;105(30):10513-89).

In a preferred embodiment, a poly-A tail is added to the miRNA. The tailed miRNA is then synthesised to cDNA by a poly-T primer with a 3' degenerate anchor and 5' universal tag. The cDNA is PCR amplified by miRNA-specific and LNA-enhanced forward and reverse primers in the presence of a dye (SYBR Green).

The amount of a miRNA may be determined by using a probe oligonucleotide that specifically detects the miRNA to be determined or of an amplification product of said miRNA.

The determination of the amount of a miRNA by specific probe oligonucleotides, preferably, comprises the step of hybridizing a miRNA or of an amplification product thereof with a probe oligonucleotide that specifically binds to the transcript or the amplification product thereof. A probe oligonucleotide in the context of the present invention, preferably, is a single-stranded nucleic acid molecule that is specific for said miRNA and, preferably, comprises a stretch of nucleotides that specifically hybridizes with the target and, thus, is complementary to the target polynucleotide. Said stretch of nucleotides is preferably 100% identical to a sequence region comprised by a target polynucleotide. The probe oligonucleotide is a preferred detection agent of the miRNAs as referred herein.

The probe oligonucleotide may be labelled or contain other modifications including enzymes which allow a determination of the amount of a miRNA (or of an amplification product thereof). Labelling can be done by various techniques well-known in the art and depending of the label to be used.

In a preferred embodiment of the method of the present invention, the determination of the amount of the miRNA comprises the step of amplifying the miRNA molecule comprised by the sample and determining the amount of the, thus, generated amplification products. By determining the amounts of said amplification products, the amount of the miRNA molecules can be assessed. Preferably, the amounts of the amplification products are determined by using probe oligonucleotides as described herein above.

If the amount of an amplification product of a miRNA shall be determined in a sample, the miRNA may be amplified as described by Mitchell et al. (loc. cit, see e.g. Fig 1a of the document Mitchell). The miRNAs are ligated at the 3' and the 5' end to single-stranded oligonucleotides that contain universal sequences for reverse transcription and for PCR. A reverse transcription of the ligation product is carried out. After reverse transcription, the resulting products are amplified by PCR. Preferably, the pair of primers that allows for specifically amplifying the miRNA biomarker, or the product of reverse transcription, is considers as detection agent as referred to herein.

In a more preferred embodiment of the method of the present invention, said PCR is a real-time PCR (RT-PCR). Thus, the amount of a miRNA is, preferably, determined by quantitative real-time PCR.

The term "real-time PCR" is known in the art. When real-time PCR is carried out, a signal emitted from the PCR assay is monitored during the PCR reaction as an indicator of the amount of amplification product during each PCR amplification cycle (as opposed to conventional PCR methods, in which the amplification product is detected at the endpoint of the PCR reaction). Real-time PCR is generally based on the detection and quantification of a fluorescent reporter. The signal increases in proportion to the amount of PCR product in a reaction.

When carrying out real-time PCR, the amount of a miRNA may be determined by comparing the results to a standard curve produced by real-time PCR of serial dilutions (e.g. undiluted, 1:10, 1:10², 1:10³, 1:10⁴, 1:10⁵, 1:10⁶, 1:10⁷) of a pre-determined amount of said marker. Moreover, to accurately determine the amount, the measured amount may be divided by the amount of normalization RNA (or DNA) which allows normalizing for possible variation of the amount of RNA between different samples. Said normalization RNA can be, preferably, RNA (or DNA) which is added to the sample (spike-in). Furthermore, one or more housekeeping miRNAs can be used as endogenous control for normalization.

### Additional determination of cytokeratin 18 fragment (M30)

Furthermore, it has been shown in the studies underlying the present invention that the additional determination of cytokeratin 18 fragment (M30) allows for a more reliable monitoring, diagnosis or assessment as referred to herein (abbreviated herein as "CK-18 (M30)").

Thus, the method for monitoring a therapy of NASH and the method for assessing whether a test compound is capable of treating NASH preferably further comprise i) the determination of the amount of the cytokeratin 18 fragment (M30) in said first and said second sample, and ii) the comparison of the amount of said cytokeratin 18 fragment (M30) in said second sample to the amount in said first sample. Based on the comparison of the amounts of the miRNA biomarkers and the amount of said cytokeratin 18 fragment (M30) in said second sample to the amounts of the miRNA biomarkers and the amount of said cytokeratin 18 fragment (M30), the treatment of NASH is monitored, or it is assessed whether the test compound is capable of treating NASH.

Thus, the method for diagnosing NASH preferably further comprises i) the determination of the amount of the cytokeratin 18 fragment (M30) in the sample from the subject, and ii) the comparison of the amount of said fragment to a reference amount for this marker. Based on i) the comparison of the amounts of the miRNA biomarkers to the reference(s) and of ii) the amount of said cytokeratin 18 fragment (M30) to the reference amount for this marker, it is diagnosed whether the test subject suffers from NASH or not.

The biomarker "cytokeratin 18 fragment (M30)" is well-known in the art.

In a preferred embodiment, the amount of the cytokeratin 18 fragment (M30) is determined by using a detection agent which specifically detects the cytokeratin 18 fragment (M30). Preferably, said detection agent is an antibody which specifically binds this marker, or antigen-binding fragment thereof. E.g., the amount of CK-18(M30) is determined by using at least one antibody which specifically binds to this marker. The at least one antibody forms a complex with the marker to determined. Afterwards, the amount of the formed complex is measured. The complex comprises the marker and the antibody (which might be labelled in order to allow for a detection of the complex).

Preferably, the antibody (or antigen binding fragment thereof) recognises a neo-epitope exposed after caspase cleavage of K18 after the aspartic acid residue 396 Cleavage at this position occurs early during apoptosis by caspase 9 and during the execution phase by caspase 3 and caspase 7. K18 is keratin 18 which is an intermediate filament protein keratin. Caspase-cleaved keratin 18 is a product of apoptosis.

In particular, the antibody or fragment thereof shall allow for determining the amount of soluble caspase-cleaved K18 (ccK18) fragments containing the K18Asp396 neo-epitope. Accordingly, the antibody specifically binds caspase-cleaved keratin 18 (ccK18, caspase-celaved cytokeratin 18, ccCK18 or K18-Asp396) but does not bind native, uncleaved, K18 Commercially available tests are e.g. based on the M30 monoclonal antibody, see Leers et al. which herewith is incorporated by reference in its entirety (Leers et al., 1999: DOI: 10.1002/(SICI)1096-9896(199904)187:5<567::AID-PATH288>3.0.CO;2-J).

The term "amount of a miRNA" as used herein encompasses the absolute amount of a miRNA (or an amplification product thereof), the relative amount or concentration thereof as well as any value or parameter which correlates thereto. Such values or parameters comprise intensity signal values from all specific physical or chemical properties obtained therefrom by direct measurements, e.g., intensity values. Moreover, encompassed are all values or parameters which are obtained by indirect measurements specified elsewhere in this description, e.g., expression levels determined from biological read out systems. It is to be understood that values correlating to the aforementioned amounts or parameters can also be obtained by all standard mathematical operations. If Real-time PCR is carried out, it is particularly preferred to determine the Ct-value (Ct: Cycle threshold) which indicates the relative amount of a marker to be analyzed in a sample. How to determine a Ct-value is well-known in the art. In general, the larger the amount of a miRNA in a sample, the lower is the Ct-value.

In an embodiment of the present invention, the amount of a miRNA biomarker as referred to herein is the copy number of the miRNA, i.e. the number of miRNAs in a given volume.

The term "amount of cytokeratin 18 fragment (M30)" as used herein encompasses the absolute amount of cytokeratin 18 fragment (M30)", the relative amount or concentration of the said biomarker as well as any value or parameter which correlates thereto or can be derived therefrom.

The term "comparing" as used herein encompasses comparing the amount of a miRNA (or of CK-18 (M30)) in a first sample with the amount of the same miRNA (or of CK-18 (M30)) in a second sample as described elsewhere herein. In the method for diagnosing, the term encompasses comparing the amount of a miRNA (or of CK-18 (M30)) in a sample from a subject who is suspected to suffer from NASH with an amount of a suitable reference specified elsewhere in this description. It is to be understood that comparing as used herein refers to a comparison of corresponding parameters or values, e.g., an absolute amount is compared to an absolute reference amount or an intensity signal obtained from a test sample is compared to the same type of intensity signal of a reference sample. The comparison referred to in the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison, it is possible to monitor a treatment of a NASH, to diagnose NASH, or to assess whether a test compound is capable of treating NASH.

For example, the method of monitoring a treatment of NASH encompasses the comparison of the amounts of said at least three biomarkers in said second sample to the amounts in the first sample. Preferably, amount of said at least three biomarkers in said second sample which differ from the amounts in said first sample are indicative for a change of the status of NASH in said subject (in particular, a change of the status in the period between the first and the second sample).

### Diagnostic algorithms

Suitable diagnostic algorithms for monitoring a treatment of NASH, for assessing whether a test compound is capable of treating NASH, or for diagnosing NASH can be established by the skilled person based on the information provided herein without further ado.

It has been shown in the studies underlying the present invention that nine elected miRNA biomarkers described herein are decreased (i.e. downregulated) in NASH patients after treatment (as compared to the amount before the treatment). This indicates that the ten of miRNA biomarkers described are increased in NASH patients as compared to patients not suffering from NASH. The nine biomarkers are indicated with an "up" in Table A .

Furthermore, it was confirmed in the studies underlying the present invention that the amount of cytokeratin 18 fragment (M30) is decreased (i.e. downregulated) in NASH patients after treatment.

**Table A: Direction of changes for miRNA and protein biomarkers**

| Biomarker | Direction of change* |
|---|---|
| CK-18(30) | up |
| miR-193b | up |
| miR-365a | up |
| miR-99a | up |
| miR-28 | up |
| let-7c | No change |
| miR-192 | up |
| miR-215 | up |
| miR-122 | up |
| miR-1260a | up |
| miR-30a | up |
| miR-34a | No change |

| | |
|---|---|
| * At baseline (M0) for no NASH vs NASH patients | |

In the following, preferred diagnostic algorithms are described.

### Method for monitoring NASH treatment:

Preferably, a decrease and, more preferably, a significant decrease, and, most preferably, a statistically significant decrease of the amount the biomarker(s) in the second sample as compared to the amount in the first sample is indicative for a subject who responds to said treatment and thus for a subject whose NASH status has improved. Preferably, said decrease is a decrease of last 20%, more preferably of at least 40%.

Also preferably, an increase and, more preferably, an significantly increase, and, most preferably, a statistically significant increase of the amount the biomarker(s) in the second sample as compared to the amount in the first sample is indicative for a subject who does not respond to said treatment and thus for a subject whose NASH status has not improved.

### Method for assessing whether a test compound is capable of treating NASH:

Preferably, a decrease and, more preferably, a significant decrease, and, most preferably, a statistically significant decrease of the amount the biomarker(s) in the second sample as compared to the amount in the first sample is indicative for a compound which is capable of treating NASH. Preferably, said decrease is a decrease of last 20%, more preferably of at least 40%.

Also preferably, an increase and, more preferably, an significantly increase, and, most preferably, a statistically significant increase of the amount the biomarker(s) in the second sample as compared to the amount in the first sample is indicative for a compound which is not capable of treating NASH. The same applies to unchanged amounts.

### Method for diagnosing NASH:

Preferably, an amount of the biomarker (or amounts of the biomarkers) which is (are) increased as compared to the reference amount for said biomarker (or the reference amounts for said biomarkers) is (are) indicative for a subject who suffers from NASH. Also preferably, an amount of the biomarker (or amounts of the biomarkers) which is (are) decreased as compared to the reference amount for said biomarker (or the reference amounts for said biomarkers) is (are) indicative for a subject who does not suffer from NASH.

As described herein above in more detail, the methods of the present invention are based on the determination of the amounts of at least three miRNA biomarkers and optionally on the determining of the amount of CK-18(M30).

Instead of comparing the individual amounts for the at least three miRNA biomarkers and optionally for CK-18(M30) to reference amounts for the at least three miRNA biomarkers and optionally for CK-18(M30), it is envisaged to calculate a single score based on the amounts of the markers.

Accordingly, the method of monitoring a NASH treatment (or the method of assessing whether a test compound is capable of treating NASH) preferably comprises i) the calculation of a first score based on the amounts of the at least three biomarkers, and optionally on the amount of CK-18(M30) in the first sample, ii) the calculation of a second score based on the amounts of the at least three biomarkers and optionally on the amount of CK-18(M30) in the second sample, and iii) the comparison of the second score to the first score. These steps are preferably carried out instead of the comparison of the amounts in step c) of the methods. Based on the comparison of the second score to the first score, the treatment is monitored, or the assessment whether a test compound is capable of treating NASH is made.

Accordingly, the method of diagnosing NASH preferably comprises i) the calculation of a score based on the amounts of the at least three biomarkers, and optionally on the amount of CK-18(M30) in the sample, and ii) the comparison of the calculated score to a reference score. These steps are preferably carried out instead of the comparison of the amounts of the markers in step b) of the method of diagnosing NASH. Based on the comparison of the calculated score to the reference score, the diagnosis of NASH is made.

The calculated scores combine information on the amounts of the at least three biomarkers and optionally on the amount of CK-18(M30). Moreover, in the scores, the biomarkers are, preferably, weighted in accordance with their contribution to the establishment of the diagnosis/monitoring/assessment. Based on the combination of biomarkers applied in the method of the invention, the weight of an individual biomarker may be different.

In a scoring system as described herein, values of different dimensions or units for the biomarkers may be used since the values will be mathematically transformed into the score.

The score can be regarded as a classifier parameter for the diagnosis/assessment or monitoring. With respect to the diagnosis of NASH, it enables the person who provides the diagnosis based on a single score based on the comparison with a reference score. The reference score is preferably a value, in particular a cut-off value which allows for differentiating between the presence of NASH and the absence of NASH in the subject to be tested. Preferably, the reference is a single value. Thus, the person does not have to interpret the entire information on the amounts of the individual biomarkers.

The definitions and explanations given herein above, preferably apply *mutatis mutandis* to the following.

The present invention further relates to the *in vitro* use of at least three biomarkers selected from the group consisting of miR-193b, miR-365a, miR-99a, miR-28, let-7c, miR-192, miR-215, and miR-122 as biomarkers, or of at least three detection agents selected from the group consisting of a detection agent which specifically detects miR-193b, a detection agent which specifically detects miR-365a, a detection agent which specifically detects miR-99a, a detection agent which specifically detects miR-28, a detection agent which specifically detects let-7c, a detection agent which specifically detects miR-192, a detection agent which specifically detects miR-215, and a detection agent which specifically detects miR-122, in a first and second sample from a subject suffering from NASH, for monitoring a treatment of NASH in said subject, wherein said first sample has been obtained from said subject before or after initiating said treatment, and wherein said second sample has been obtained from said subject after said first sample.

The present invention further relates to the *in vitro* use of at least three biomarkers selected from the group consisting of miR-193b, miR-365a, miR-99a, miR-28, let-7c, miR-192, miR-215, and miR-122 as a biomarker, or of at least three detection agents selected from the group consisting of a detection agent which specifically detects miR-193b, a detection agent which specifically detects miR-365a, a detection agent which specifically detects miR-28, a detection agent which specifically detects let-7c, a detection agent which specifically detects miR-192, a detection agent which specifically detects miR-215, and a detection agent which specifically detects miR-122, in a sample from a subject suspected to suffer from NASH for diagnosing whether said subject suffers from NASH.

The present invention further relates to the *in vitro* use of at least three biomarkers selected from the group consisting of miR-193b, miR-365a, miR-99a, miR-28, let-7c, miR-192, miR-215, and miR-122 as a biomarker, or of at least three detection agents selected from the group consisting of a detection agent which specifically detects miR-193b, a detection agent which specifically detects miR-365a, a detection agent which specifically detects miR-99a, a detection agent which specifically detects miR-28, a detection agent which specifically detects let-7c, a detection agent which specifically detects miR-192, a detection agent which specifically detects miR-215, and a detection agent which specifically detects miR-122, in a first and second sample from a subject suffering from NASH for assessing whether a test compound is capable of treating NASH, wherein said first sample has been obtained from said subject before or after initiating administration of said test compound, and wherein said second sample has been obtained after said first sample.

The aforementioned uses may further comprise the use
- of cytokeratin 18 fragment (M30) as a biomarker, or
- of a detection agent which specifically detects the cytokeratin 18 fragment (M30),
- and/or
- of at least one further miRNA biomarker selected from the group consisting of miR-1260a, miR-30a, and miR-34a, or
- of at least one detection agent selected from the group consisting of a detection agent which specifically detects miR-1260a, a detection agent which specifically detects miR-30a, a detection agent which specifically detects miR-34a.

In addition, the present invention relates to composition comprising at least three detection agents selected from the group consisting of a detection agent which specifically detects miR-193b, a detection agent which specifically detects miR-365a, a detection agent which specifically detects miR-99a, a detection agent which specifically detects miR-28, a detection agent which specifically detects let-7c, a detection agent which specifically detects miR-192, a detection agent which specifically detects miR-215, and a detection agent which specifically detects miR-122.

Finally, the present invention relates to a kit comprising at least three detection agents selected from the group consisting of a detection agent which specifically detects miR-193b, a detection agent which specifically detects miR-365a, a detection agent which specifically detects miR-99a, a detection agent which specifically detects miR-28, a detection agent which specifically detects let-7c, a detection agent which specifically detects miR-192, a detection agent which specifically detects miR-215, and a detection agent which specifically detects miR-122.

The kit and the composition may further comprise a detection agent which specifically detects the cytokeratin 18 fragment (M30) and/or at least one detection agent selected from the group consisting of a detection agent which specifically detects miR-1260a, a detection agent which specifically detects miR-30a, a detection agent which specifically detects miR-34a.

The term "kit" as used herein refers to a collection of the aforementioned components, preferably, provided separately or within a single container. The detection agents may be provided in the kit of the invention in a "ready-to-use" form. The kit may further include controls, buffers, and/or reagents. The kit also comprises instructions for carrying out the method of the present invention. These instructions may be in the form of a manual or may be electronically accessible information. The latter information may be provided on a data storage medium or device such as an optical storage medium (e.g., a Compact Disc) or directly on a computer or data processing device.

In the following, embodiments of the present inventions are disclosed. The definitions and explanations given herein above apply *mutatis mutandis.*
1. A method for monitoring a treatment of non-alcoholic steatohepatitis (NASH) in a subject suffering from NASH, comprising
   (a) determining the amounts of at least three biomarkers selected from the group consisting of miR-193b, miR-365a, miR-99a, miR-28, let-7c, miR-192, miR-215, and miR-122 in a first sample which has been obtained from said subject before or after initiating said treatment,
   (b) determining the amounts of said at least three biomarkers in a second sample which has been obtained from said subject after said first sample, and
   (c) comparing the amounts of said at least three biomarkers in said second sample to the amounts of said at least three biomarkers in said first sample, thereby monitoring the treatment of NASH.
2. The method of embodiment 1, wherein said subject is human.
3. The method of embodiments 1 and 2, wherein said sample is a blood, serum or plasma sample.
4. The method any one of embodiments 1 to 3, wherein said second sample has been obtained at least one month after said first sample.
5. The method of any one of embodiments 1 to 4, wherein the amounts of the at least three biomarkers in steps a) and b) are determined by RT-PCT.
6. The method of embodiment 5, wherein said RT-PCR comprises (i) enriching miRNAs from said first and said second sample, (ii) carrying out a reverse transcriptase reaction on the enriched miRNAs, thereby producing cDNA transcripts corresponding to the miRNAs, (iii) amplifying the produced cDNA transcripts by polymerase chain reaction with a primer pair which allows for specifically detecting the biomarker, and (iv) quantifying the amplified cDNA transcripts.
7. The method according to any one of embodiments 1 to 6, wherein amounts of said at least three biomarkers in said second sample which differ from the amounts in said first sample are indicative for a change of the status of NASH in said subject.
8. The method of any one of embodiments 1 to 6, wherein the comparison of the amounts in step c) comprises i) the calculation of a first score based on the amounts of the at least three biomarkers in the first sample, ii) the calculation of a second score based on the amounts of the at least three biomarkers in the second sample, and iii) the comparison of the second score to the first score.
9. The method of any one of embodiments 1 to 8, wherein said subject suffers from diabetes mellitus, in particular from type 2 diabetes mellitus.
10. The method of any one of embodiments 1 to 9, wherein said subject has a body mass index of larger than 30.
11. The method of any one of embodiments 1 to 10, wherein said treatment of NASH is gastric bypass surgery.
12. The method of any one of embodiments 1 to 10, wherein said treatment of NASH is a drug-based treatment.
13. The method of embodiment 12, wherein said drug-based treatment comprises the administration of at least one drug selected from statins, incretin analogues, metformin, rimonabant, thiazolidinediones, and orlistat.
14. The method of any one of embodiments 1 to 13, further comprising i) the determination of the amount of the cytokeratin 18 fragment (M30) in said first and said second sample, and ii) the comparison of the amount of said fragment in said second sample to the amount in said first sample.
15. The method of embodiment 14, wherein the amount of the cytokeratin 18 fragment (M30) is determined by using a detection agent which specifically detects the cytokeratin 18 fragment (M30) such as an antibody, or antigen-binding fragment thereof.
16. The method of any one embodiments 1 to 15, wherein miR-193b is one of the at least three biomarkers.
17. The method of any one of embodiments 1 to 16, wherein the biomarkers miR-193b and miR-365a are two of the at least three biomarkers.
18. The method of any one of embodiments 1 to 17, further comprising i) the determination of the amount of at least one further miRNA biomarker in said first and said second sample, wherein said at least one further miRNA biomarker is selected from the group consisting of miR-1260a, miR-30a, and miR-34a, and ii) the comparison of the amount of said at least one further miRNA biomarker in said second sample to the amount in said first sample.
19. A method for diagnosing non-alcoholic steatohepatitis (NASH) in a subject suspected to suffer from NASH, comprising
   (a) determining the amounts of at least three biomarkers selected from the group consisting of miR-193b, miR-365a, miR-99a, miR-28, let-7c, miR-192, miR-215, and miR-122 in a sample from said subject, and
   (b) comparing the amounts of said at least three biomarkers to a reference, thereby diagnosing NASH.
20. The method of embodiment 19, wherein the comparison of the amounts of said at least three biomarkers to a reference in step b) comprises the comparison of a score calculated based on the amounts of said at least three biomarkers to a reference score.
21. A method for assessing whether a test compound is capable of treating NASH, comprising:
   (a) determining the amounts of at least three biomarkers selected from the group consisting of miR-193b, miR-365a, miR-99a, miR-28, let-7c, miR-192, miR-215, and miR-122 in a first sample which has been obtained from a subject suffering from NASH before or after initiating administration of said test compound,
   (b) determining the amounts of said at least three biomarkers in a second sample which has been obtained from said subject after said first sample, and
   (c) comparing the amounts of said at least three biomarkers in said second sample to the amount of said at least three biomarkers in said first sample, thereby assessing whether said test compound is capable of treating NASH.
22. *In vitro* use of at least three biomarkers selected from the group consisting of miR-193b, miR-365a, miR-99a, miR-28, let-7c, miR-192, miR-215, and miR-122 as biomarkers, or of at least three detection agents selected from the group consisting of a detection agent which specifically detects miR-193b, a detection agent which specifically detects miR-365a, a detection agent which specifically detects miR-99a, a detection agent which specifically detects miR-28, a detection agent which specifically detects let-7c, a detection agent which specifically detects miR-192, a detection agent which specifically detects miR-215, and a detection agent which specifically detects miR-122, in a first and second sample from a subject suffering from NASH, for monitoring a treatment of NASH in said subject, wherein said first sample has been obtained from said subject before or after initiating said treatment, and wherein said second sample has been obtained from said subject after said first sample.
23. *In vitro* use of at least three biomarkers selected from the group consisting of miR-193b, miR-365a, miR-99a, miR-28, let-7c, miR-192, miR-215, and miR-122 as a biomarker, or of at least three detection agents selected from the group consisting of a detection agent which specifically detects miR-193b, a detection agent which specifically detects miR-365a, a detection agent which specifically detects miR-28, a detection agent which specifically detects let-7c, a detection agent which specifically detects miR-192, a detection agent which specifically detects miR-215, and a detection agent which specifically detects miR-122, in a sample from a subject suspected to suffer from NASH for diagnosing whether said subject suffers from NASH.
24. *In vitro* use of at least three biomarkers selected from the group consisting of miR-193b, miR-365a, miR-99a, miR-28, let-7c, miR-192, miR-215, and miR-122 as a biomarker, or of at least three detection agents selected from the group consisting of a detection agent which specifically detects miR-193b, a detection agent which specifically detects miR-365a, a detection agent which specifically detects miR-99a, a detection agent which specifically detects miR-28, a detection agent which specifically detects let-7c, a detection agent which specifically detects miR-192, a detection agent which specifically detects miR-215, and a detection agent which specifically detects miR-122, in a first and second sample from a subject suffering from NASH for assessing whether a test compound is capable of treating NASH, wherein said first sample has been obtained from said subject before or after initiating administration of said test compound, and wherein said second sample has been obtained after said first sample.
25. The *in vitro* use of any one of embodiments 20 to 22, further comprising the use of cytokeratin 18 fragment (M30) as a biomarker, or of a detection agent which specifically detects the cytokeratin 18 fragment (M30).
26. The *in vitro* use of any one of embodiments 22 to 25, further comprising the use of at least one further miRNA biomarker selected from the group consisting of miR-1260a, miR-30a, and miR-34a, or of at least one detection agent which specifically detects said at least one further miRNA biomarker.
27. A composition comprising at least three detection agents selected from the group consisting of a detection agent which specifically detects miR-193b, a detection agent which specifically detects miR-365a, a detection agent which specifically detects miR-99a, a detection agent which specifically detects miR-28, a detection agent which specifically detects let-7c, a detection agent which specifically detects miR-192, a detection agent which specifically detects miR-215, and a detection agent which specifically detects miR-122.
28. A kit comprising at least three detection agents selected from the group consisting of a detection agent which specifically detects miR-193b, a detection agent which specifically detects miR-365a, a detection agent which specifically detects miR-99a, a detection agent which specifically detects miR-28, a detection agent which specifically detects let-7c, a detection agent which specifically detects miR-192, a detection agent which specifically detects miR-215, and a detection agent which specifically detects miR-122.
29. The kit of embodiment 28, further comprising a detection agent which specifically detects the cytokeratin 18 fragment (M30).

All references cited herein are herewith incorporated by reference with respect to their disclosure content in general or with respect to the specific disclosure contents indicated above.

The invention will now be illustrated by the following Examples which are not intended to restrict or limit the scope of this invention.

### EXAMPLES

### Materials and Methods

### Subjects

The study included 74 obese patients (34.8 ≤ BMI ≤ 71.3) with T2D (5 ≤ HbAlc ≤ 12.2), whose undergo a bariatric surgery. A significant weight loss was observed three months after the surgery (mean change in weight of -19.22 ± 2.2 kg). The study population consisted of 47 females and 27 males with age ranges from 27 to 67 years (mean = 50.4 ±9.3). Many parameters such as demographic characteristics; diabetic family history; family history of obesity; were collected at screening. Clinical data were longitudinally collected before surgery; 1 month; 3 months and 12 months after a bariatric surgery. Liver parameters of these patients were at baseline in mean AST of 28.3 (±12.7) IU/l, ALT of 36.7 (±23.0) IU/l and GGT of 66.4 (±73.7) IU/l. Liver histology was performed before surgery allowing the measurement of several parameters (fibrosis status, steatosis, inflammation, ballooning and NAS) that were used to derive a NASH status (yes/no) for each patient. The patients were divided into two groups: no NASH (control) group (n=54) and NASH group (n=20) based on histological parameters. NASH is defined as fibrosis score ≥ 3 or/and ballooning score ≥ 1.

An overview on the patients is provided in the following table.

| | **Baseline** | | **3 months after surgery** | |
|---|---|---|---|---|
| | **no NASH** | **NASH** | **no NASH** | **NASH** |
| **Age** | **50 (±20)** | **51 (±23)** | | |
| **Sex** | **f (35);m (19)** | **f(12); m(8)** | | |
| **BMI** | **45.5** (±6.5) | **44.5** (±8.2) | **38.52** (±6.33) | **37.46** (±6.72) |
| **HbA1c** | **7.34** (±1.50) | **8.13** (±1.83) | **6.14** (±1.00) | **6.08** (±1.08) |
| **AST** | **26.21** (±11.93) | **34.00** (±13.19) | **29.77** (±13.47) | **32.22** (±9.94) |
| **ALT** | **33.35** (±21.54) | **45.90** (±25.02) | **30.92** (±14.24) | **34.39** (±12.47) |
| **GGT** | **51.56** (±49.13) | **106.65** (±108.53) | **23.24** (+16.55) | **41.70** (±39.75) |

### miRNA measurement: Isolation and Analysis

Serum RNA before and three months after surgery was isolated using miRNeasy Serum/ Plasma Kit provided by Qiagen (Hilden, Germany) according to the manufacturer's instructions. Briefly, we started with 2 aliquots of 250 µl serum per patient, performed phenol/chloroform extraction and loaded both aliquots on one column. After three washing steps RNA was eluted in 14 µl H₂O.

The cDNA synthesis and qPCR of ∼681 miRNAs was performed with the miRCURY LNA™ Universal RT miRNA PCR System by Exiqon (Vedbaek, Denmark). All miRNas were polyandenylated and reverse transcribed into cDNA in a single reaction. cDNA and ExiLENT SYBR Green master mix were transferred to qPCR panels pre-loaded with primers, using pipetting robot. Amplification is performed in a Roche lightcycler 480 (Basel, Switzerland).

### CK18 (M30) measurement

The apoptosis-associated neoepitope CK18 (M30) was measured by using the M30-Apoptosense ELISA provided by Peviva (Bromma, Sweden) according to the manufacturer's instructions.

### Data Analysis

In total 164 miRNAs passed the QC. Only those miRNAs with a cut-off of raw Ct < 35 in at least 50% of all samples were analyzed. The miRNA levels were normalized to the mean of 72 miRNAs (global normalization) only taking into account miRNAs with Ct < 35. The relative expression was calculated using 2^{-ΔCT}. Missing values were imputed by the relative expression level by 50% of the lowest relative expression level of this miRNA across all patients by visit. Fold changes comparing 3 months versus baseline are reported and fold change baseline no NASH versus NASH. p-values are the false discovery rate (FDR) adjusted as proposed by Benjamini-Hochberg. Correlation analyses were performed by Spearman correlation between CK-18, each clinical liver and histological liver parameter with all miRNAs. ROC analyses were computed to evaluate the diagnostic power. Sensitivity and specificity was determined by maximizing the Youden index.

### Results

### Differential expression of circulating miRNAs before and after bariatric surgery

Diagnosis of non-alcoholic steatohepatitis (NASH) patients is crucial, as they are at a high risk for cirrhosis due to the progression of fibrosis. Elevated clinical liver parameter levels lack specificity for NASH. The only reliable diagnosis by liver biopsy is invasive and risky. Therefore we worked on the identification of a non-invasive biomarker panel for NASH progression and diagnosis.

miRNAs are 21nt long, non-coding RNAs, which regulate gene expression at the posttran-scriptional level. miRNAs that are released into the blood circulation are bound to microparticles. They are highly stable in liquid biopsies and are promising candidates as new prognostic and diagnostic biomarkers.

NASH can be treated with bariatric surgery. We used serum samples of 74 obese T2D patients before and 3 months after surgery to identify markers related to NASH improvement. Over 650 miRNAs were profiled of which 164 miRNAs were found in quantifiable concentrations. After surgery 10 miRNAs and the known NASH biomarker Cytokeratin-18 (CK-18, M30 fragment) were significantly decreased (Table 1).

**Tab.1: Significant downregulated miRNAs and CK-18 after bariatric surgery. The fold change of three months after surgery miRNA/CK-18 levels versus baseline miR-NA/CK-18 levels was calculated. All 10 miRNAs and CK-18 are significantly downregulated.**

| **miRNA** | **fold change** | **p-value** |
|---|---|---|
| miR-193b-3p | -2.71 | 0.000328 |
| miR-122-5p | -1.95 | 0.003953 |
| miR-193a-5p | -1.90 | 0.000034 |
| miR-365a-3p | -1.57 | 0.003681 |
| miR-30a-5p | -1.57 | 0.000034 |
| miR-125b-5p | -1.49 | 0.009686 |
| miR-194-5p | -1.47 | 0.000001 |
| miR-99a-5p | -1.44 | 0.003614 |
| miR-941 | -1.43 | 0.000160 |
| miR-192-5p | -1.41 | 0.000014 |
| **protein** | | |
| CK-18 (M30) | -1.80 | 0.000066 |

### Correlation of apoptosis depended CK-18, liver and histological parameters with circulating serum miRNA levels

So far, liver biopsy is the only method to reliably diagnose NASH. Therefore there is a major need to reduce patient burden by using non-invasive biomarkers for NASH diagnosis and for prediction of NASH progression. Individual markers have been identified that correlate with NAFLD and NASH, however, none of them is specific. Since NASH is defined by a combination of changes in the tissue, using a combination of markers - a panel - to capture several characteristics and distinguish NASH from other liver diseases will have better chances of success.

To identify a biomarker panel for NASH diagnosis we used the NASH marker CK-18 (M30 fragment) (Tab. 2 and 3), clinical parameters (e.g. AST, ALT and GGT) (Tab.4-6) and histological parameters from liver biopsies (Tab. 7 and 8) for correlational analyses with serum miRNA levels.

**Tab.2: Correlation analysis of CK-18 levels with miRNA levels. 20 best correlated miRNAs with serum CK-18 at baseline. Bold are all miRNAs that are included in a NASH biomarker panel of 12 miRNAs (and CK-18).**

| **miRNA** | **p-values of correlation with CK18** |
|---|---|
| **hsa-miR-193b-3p** | 9.2159E-11 |
| **hsa-miR-365a-3p** | 9.3421E-09 |
| hsa-miR-885-5p | 3.0729E-07 |
| **hsa-miR-99a-5p** | 4.6287E-07 |
| **hsa-miR-193a-5p** | 6.1701E-07 |
| **hsa-miR-122-5p** | 6.2506E-07 |
| **hsa-miR-34a-5p** | 3.7659E-06 |
| **hsa-miR-192-5p** | 8.7793E-06 |
| hsa-miR-125b-5p | 2.6635E-05 |
| mmu-miR-378a-3p | 5.2088E-05 |
| **hsa-miR-215-5p** | 6.8133E-05 |
| hsa-miR-505-3p | 0.0001028 |
| hsa-miR-100-5p | 0.00046044 |
| hsa-let-7b-3p | 0.00052625 |
| hsa-miR-483-5p | 0.00071343 |
| **hsa-miR-30a-5p** | 0.00087348 |
| hsa-miR-194-5p | 0.00090947 |
| hsa-miR-27b-3p | 0.00155885 |
| hsa-miR-21-5p | 0.00248802 |
| hsa-miR-148a-3p | 0.00367243 |

Since it is known that bariatric surgery (Roux-en-Y gastric bypass) improves NASH status significantly, we calculated the fold changes of CK-18 (M30 fragment) and miRNAs levels before and three months after surgery and subsequently performed correlational analysis with the surgery induced change (Tab.3). Therefore our panel contains dynamic information related to NASH improvement after bariatric surgery as well as contains biomarkers that correlate with liver histology parameters (Tab. 7 and 8).

**Tab.3: Correlation analysis of CK-18 levels change after surgery with miRNA levels change. 20 best correlated miRNAs changes with CK-18 change upon surgery. Bold are all miRNAs that are included to the NASH biomarker panel.**

| **miRNA** | **p-values of correlation with CK18 change vs. miRNA change** |
|---|---|
| **hsa-miR-365a-3p** | 1.43806E-05 |
| **hsa-miR-193b-3p** | 1.89825E-05 |
| hsa-miR-193a-5p | 0.000249516 |
| **hsa-miR-99a-5p** | 0.000396175 |
| hsa-miR-885-5p | 0.00068746 |
| **hsa-miR-192-5p** | 0.001550757 |
| **hsa-miR-122-5p** | 0.002379159 |
| hsa-miR-505-3p | 0.003976111 |
| hsa-miR-140-5p | 0.004182791 |
| hsa-miR-194-5p | 0.005187818 |
| **hsa-miR-215-5p** | 0.009586269 |
| hsa-miR-22-3p | 0.010140391 |
| hsa-miR-125b-5p | 0.018954994 |
| hsa-miR-26b-5p | 0.020679924 |
| hsa-miR-22-5p | 0.023976503 |
| hsa-miR-483-5p | 0.026312474 |
| hsa-miR-455-3p | 0.038816765 |
| **hsa-miR-30a-5p** | 0.040445039 |
| hsa-miR-100-5p | 0.042437215 |
| hsa-miR-210-3p | 0.045197265 |

**Tab.4: Correlation analysis of AST with miRNA levels. 20 best correlated miRNAs with AST at baseline. Bold are all miRNAs that are included to the NASH biomarker panel. Listed are the correlation coefficient, p-value, Benjamini Hochberg adjusted p-value and the correlation method.**

| **miRNA** | **correlation coefficient** | **p-value** | **BH adjusted p-value** | **Method** |
|---|---|---|---|---|
| **hsa-miR-365a-3p** | 0.513 | 2.1747E-06 | 0.00027628 | Spearman |
| **hsa-miR-193b-3p** | 0.505 | 3.3692E-06 | 0.00027628 | Spearman |
| hsa-miR-193a-5p | 0.47 | 1.8773E-05 | 0.00102626 | Spearman |
| **hsa-miR-99a-5p** | 0.447 | 5.2497E-05 | 0.00189186 | Spearman |
| hsa-miR-885-5p | 0.444 | 5.7679E-05 | 0.00189186 | Spearman |
| **hsa-miR-192-5p** | 0.438 | 7.5638E-05 | 0.00206745 | Spearman |
| **hsa-miR-122-5p** | 0.419 | 0.00016506 | 0.00386703 | Spearman |
| hsa-miR-505-3p | 0.412 | 0.00021963 | 0.00450246 | Spearman |
| hsa-miR-140-5p | 0.401 | 0.00033244 | 0.00605783 | Spearman |
| hsa-miR-194-5p | 0.388 | 0.00052854 | 0.00866808 | Spearman |
| **hsa-miR-215-5p** | 0.383 | 0.00063048 | 0.00939995 | Spearman |
| hsa-miR-22-3p | -0.365 | 0.0011852 | 0.01619779 | Spearman |
| hsa-miR-125b-5p | 0.316 | 0.00536463 | 0.06767682 | Spearman |
| hsa-miR-26b-5p | 0.299 | 0.00862448 | 0.10102964 | Spearman |
| hsa-miR-22-5p | 0.294 | 0.0098326 | 0.10750309 | Spearman |
| hsa-miR-483-5p | -0.289 | 0.01136448 | 0.11648591 | Spearman |
| hsa-miR-455-3p | -0.276 | 0.01584851 | 0.1528915 | Spearman |
| **hsa-miR-30a-5p** | 0.269 | 0.01871309 | 0.17049709 | Spearman |
| hsa-miR-100-5p | 0.257 | 0.02480108 | 0.21407247 | Spearman |
| hsa-miR-210-3p | -0.25 | 0.02959016 | 0.24263931 | Spearman |

**Tab.5: Correlation analysis of ALT with miRNA levels. 20 best correlated miRNAs with ALT at baseline. Bold are all miRNAs that are included to the NASH biomarker panel. Listed are the correlation coefficient, p-value, Benjamini Hochberg adjusted p-value and the correlation method.**

| **miRNA** | **correlation coefficient** | **p-value** | **BH adjusted p-value** | **Method** |
|---|---|---|---|---|
| **hsa-miR-193b-3p** | 0.552 | 1.9175E-07 | 1.9373E-05 | Spearman |
| **hsa-miR-34a-5p** | 0.549 | 2.3626E-07 | 1.9373E-05 | Spearman |
| hsa-miR-885-5p | 0.52 | 1.2374E-06 | 6.7643E-05 | Spearman |
| hsa-miR-193a-5p | 0.504 | 2.928E-06 | 0.00012005 | Spearman |
| **hsa-miR-122-5p** | 0.49 | 6.1925E-06 | 0.00020311 | Spearman |
| **hsa-miR-192-5p** | 0.424 | 0.00012225 | 0.00250613 | Spearman |
| **hsa-miR-365a-3p** | 0.424 | 0.00012094 | 0.00250613 | Spearman |
| **hsa-miR-99a-5p** | 0.424 | 0.00011995 | 0.00250613 | Spearman |
| **hsa-miR-30a-5p** | 0.405 | 0.000257 | 0.00468303 | Spearman |
| hsa-miR-125b-5p | 0.393 | 0.0003998 | 0.00655671 | Spearman |
| **hsa-miR-1260a** | 0.379 | 0.00067391 | 0.009678 | Spearman |
| hsa-miR-424-5p | -0.378 | 0.00070815 | 0.009678 | Spearman |
| **hsa-miR-215-5p** | 0.365 | 0.00111514 | 0.01406789 | Spearman |
| hsa-miR-483-3p | 0.338 | 0.00262401 | 0.03073835 | Spearman |
| hsa-miR-194-5p | 0.311 | 0.00584429 | 0.06389758 | Spearman |
| hsa-miR-483-5p | 0.281 | 0.01315871 | 0.13487674 | Spearman |
| hsa-miR-148a-3p | 0.272 | 0.0168524 | 0.16257605 | Spearman |
| hsa-miR-100-5p | 0.267 | 0.01876576 | 0.17097691 | Spearman |
| hsa-miR-629-5p | -0.24 | 0.03534872 | 0.30511528 | Spearman |
| hsa-miR-505-3p | 0.236 | 0.03850406 | 0.31573332 | Spearman |

**Tab.6: Correlation analysis of GGT with miRNA levels. 20 best correlated miRNAs with GGT at baseline. Bold are all miRNAs that are included to the NASH biomarker panel. Listed are the correlation coefficient, p-value, Benjamini Hochberg adjusted p-value and the correlation method.**

| **miRNA** | **correlation coefficient** | **p-value** | **BH adjusted p-value** | **Method** |
|---|---|---|---|---|
| **hsa-miR-193b-3p** | 0.443 | 5.4149E-05 | 0.00888046 | Spearman |
| **hsa-miR-192-5p** | 0.412 | 0.00020016 | 0.01641276 | Spearman |
| **hsa-miR-1260a** | 0.397 | 0.00035179 | 0.01923109 | Spearman |
| hsa-miR-193a-5p | 0.349 | 0.00184156 | 0.05085882 | Spearman |
| **hsa-miR-34a-5p** | 0.342 | 0.0023229 | 0.05085882 | Spearman |
| **hsa-miR-365a-3p** | 0.34 | 0.00245933 | 0.05085882 | Spearman |
| hsa-miR-885-5p | 0.34 | 0.00248092 | 0.05085882 | Spearman |
| **hsa-miR-99a-5p** | 0.34 | 0.00245475 | 0.05085882 | Spearman |
| **hsa-miR-122-5p** | 0.313 | 0.00559286 | 0.1019143 | Spearman |
| **hsa-miR-215-5p** | 0.299 | 0.00830134 | 0.13614194 | Spearman |
| hsa-let-7i-5p | -0.276 | 0.01529079 | 0.20158521 | Spearman |
| hsa-miR-505-3p | 0.276 | 0.01499812 | 0.20158521 | Spearman |
| **hsa-miR-30a-5p** | 0.274 | 0.01597932 | 0.20158521 | Spearman |
| hsa-miR-483-5p | 0.267 | 0.01911218 | 0.22388554 | Spearman |
| hsa-miR-194-5p | 0.263 | 0.02073324 | 0.22668348 | Spearman |
| hsa-miR-455-3p | 0.255 | 0.02501396 | 0.2455414 | Spearman |
| hsa-miR-629-5p | -0.255 | 0.02545246 | 0.2455414 | Spearman |
| hsa-miR-378a-5p | 0.246 | 0.03104713 | 0.28287387 | Spearman |
| hsa-miR-15b-5p | -0.236 | 0.03902557 | 0.31859185 | Spearman |
| hsa-miR-15a-5p | -0.234 | 0.04041665 | 0.31859185 | Spearman |

**Tab.7: Correlation analysis of steatosis, ballooning and lobular inflammation with miRNA levels. The 20 best correlated miRNAs with steatosis, ballooning and lobular inflammation at baseline. Bold are all miRNAs that are included to the NASH biomarker panel.**

| **miRNA** | **Steatosis (p-value)** | **miRNA** | **Ballooning (p-value)** | **miRNA** | **Lobular Inflammation (p-value)** |
|---|---|---|---|---|---|
| **hsa-miR-365a-3p** | 0.001645 | **hsa-miR-28-3p** | 0.007922 | **hsa-let-7c-5p** | 0.000299 |
| **hsa-miR-1260a** | 0.002858 | **hsa-miR-1260a** | 0.012601 | hsa-miR-148b-3p | 0.000369 |
| **hsa-miR-193b-3p** | 0.005407 | hsa-miR-193a-5p | 0.029048 | hsa-let-7a-5p | 0.002089 |
| **hsa-miR-192-Sp** | 0.010318 | hsa-miR-140-5p | 0.030335 | **hsa-miR-193b-3p** | 0.002139 |
| hsa-miR-193a-5p | 0.011622 | hsa-miR-501-3p | 0.047062 | hsa-miR-629-5p | 0.002306 |
| hsa-miR-151a-3p | 0.012558 | hsa-miR-143-3p | 0.050108 | **hsa-miR-30a-5p** | 0.002819 |
| **hsa-miR-215-5p** | 0.013394 | hsa-miR-342-5p | 0.052934 | hsa-miR-142-3p | 0.003104 |
| hsa-miR-2110 | 0.018099 | hsa-let-7f-5p | 0.059388 | hsa-miR-125b-5p | 0.004568 |
| hsa-miR-624-5p | 0.027213 | hsa-miR-331-3p | 0.065226 | **hsa-miR-122-5p** | 0.007075 |
| hsa-miR-885-5p | 0.037257 | hsa-miR-125a-5p | 0.076923 | hsa-miR-30b-5p | 0.010084 |
| hsa-miR-629-5p | 0.040332 | **hsa-miR-34a-5p** | 0.078989 | hsa-miR-19a-3p | 0.013417 |
| hsa-miR-501-3p | 0.041146 | hsa-miR-151a-5p | 0.092803 | hsa-miR-296-5p | 0.018992 |
| hsa-miR-320c | 0.042816 | hsa-miR-99b-5p | 0.094833 | hsa-miR-194-5p | 0.019432 |
| hsa-miR-30d-5p | 0.047463 | hsa-miR-17-3p | 0.110647 | hsa-miR-103a-3p | 0.021464 |
| hsa-miR-342-3p | 0.051085 | hsa-miR-423-3p | 0.127854 | **hsa-miR-365a-3p** | 0.024087 |
| hsa-miR-663b | 0.057780 | hsa-miR-30e-5p | 0.134986 | **hsa-miR-99a-5p** | 0.025149 |
| hsa-miR-148b-3p | 0.059133 | **hsa-miR-193b-3p** | 0.148294 | **hsa-miR-192-Sp** | 0.025407 |
| hsa-miR-148a-3p | 0.065754 | hsa-miR-296-5p | 0.153740 | **hsa-miR-1260a** | 0.026039 |
| **hsa-let-7c-5p** | 0.067999 | hsa-miR-30d-5p | 0.159487 | hsa-miR-660-5p | 0.030627 |
| hsa-miR-130a-3p | 0.074133 | hsa-miR-326 | 0.174856 | hsa-miR-7-1-3p | 0.030933 |

**Tab.8: Correlation analysis of fibrosis score, NAS and NASH status with miRNA levels. The 20 best correlated miRNAs with fibrosis score, NAS and NASH status at baseline. Bold are all miRNAs that are included to the NASH biomarker panel.**

| **miRNA** | **Fibrosis score (p-value)** | **miRNA** | **NAS (p-value)** | **miRNA** | **NASH status (p-value)** |
|---|---|---|---|---|---|
| **hsa-miR-193b-3p** | 0.000053 | **hsa-miR-1260a** | 0.000450 | **hsa-miR-1260a** | 0.005044 |
| **hsa-miR-99a-5p** | 0.000312 | **hsa-miR-193b-3p** | 0.000470 | hsa-miR-193a-5p | 0.006149 |
| **hsa-miR-192-Sp** | 0.000368 | **hsa-miR-365a-3p** | 0.001479 | **hsa-miR-28-3p** | 0.008571 |
| **hsa-miR-365a-3p** | 0.000440 | hsa-miR-148b-3p | 0.002907 | hsa-miR-501-3p | 0.009630 |
| **hsa-miR-34a-5p** | 0.000520 | hsa-miR-193a-5p | 0.003421 | **hsa-miR-34a-5p** | 0.012698 |
| hsa-miR-193a-5p | 0.000621 | hsa-miR-501-3p | 0.006603 | **hsa-miR-193b-3p** | 0.025669 |
| **hsa-miR-122-5p** | 0.000621 | **hsa-let-7c-5p** | 0.010506 | hsa-miR-331-3p | 0.030564 |
| hsa-miR-125b-5p | 0.000625 | **hsa-miR-192-Sp** | 0.014099 | hsa-let-7f-5p | 0.051542 |
| hsa-miR-30a-5p | 0.000669 | hsa-miR-629-5p | 0.014388 | hsa-miR-143-3p | 0.063550 |
| hsa-miR-194-5p | 0.002388 | **hsa-miR-34a-5p** | 0.017956 | hsa-miR-140-5p | 0.072741 |
| hsa-miR-100-5p | 0.002664 | hsa-miR-885-5p | 0.018479 | **hsa-miR-365a-3p** | 0.081780 |
| **hsa-miR-215-5p** | 0.002877 | hsa-miR-19a-3p | 0.019491 | hsa-miR-424-5p | 0.091101 |
| hsa-miR-7-1-3p | 0.008425 | hsa-miR-186-5p | 0.023299 | hsa-miR-342-5p | 0.105432 |
| hsa-miR-885-5p | 0.014682 | **hsa-miR-215-5p** | 0.024775 | hsa-miR-10b-5p | 0.131166 |
| hsa-miR-505-3p | 0.015293 | hsa-miR-296-5p | 0.027120 | **hsa-miR-99a-5p** | 0.133937 |
| hsa-miR-501-3p | 0.016288 | **hsa-miR-99a-5p** | 0.027872 | hsa-miR-125a-5p | 0.154265 |
| hsa-miR-574-3p | 0.025359 | hsa-let-7a-5p | 0.029019 | hsa-miR-885-5p | 0.164607 |
| mmu-miR-378a-3p | 0.028782 | **hsa-miR-122-5p** | 0.038210 | hsa-miR-26b-5p | 0.165469 |
| hsa-miR-143-3p | 0.030033 | hsa-miR-125b-5p | 0.040201 | **hsa-miR-192-Sp** | 0.165499 |
| **hsa-miR-1260a** | 0.030535 | hsa-miR-30b-5p | 0.042265 | hsa-miR-197-3p | 0.171373 |

Based on the analysis above, a panel of 12 biomarkers including miR-193b, miR-365a, miR-99a, miR-192, miR-122, miR-28, miR-1260a, miR-34a, let-7c, miR-215, and miR-30a was established.

### Diagnostic performance of routine liver parameters, CK18 (M30) fragments and miRNA levels in circulation

A preliminary evaluation of the diagnostic power of a panel of the 12 miRNAs (and optionally CK-18) was done by ROC curve analysis. We demonstrated that the established NASH biomarker panel showed better performance for NASH diagnosis than known liver biomarkers like transaminases.

**Tab.9: ROC analysis of different variables for diagnostic power.**

| **Independent variables** | **Max AUC** |
|---|---|
| CK-18 | 0.70 |
| Liver parameter | 0.70 |
| CK-18 & liver parameter | 0.68 |
| miRNA panel | 0.72 |
| miRNA panel & CK-18 | 0.73 |
| miRNA panel & liver parameter | 0.68 |
| miRNA panel & CK-18 & liver parameter | 0.67 |

## Claims

1. A method for monitoring a treatment of non-alcoholic steatohepatitis (NASH) in a subject suffering from NASH, comprising
(a) determining the amounts of at least three biomarkers selected from the group consisting of miR-193b, miR-365a, miR-99a, miR-28, let-7c, miR-192, miR-215, and miR-122 in a first sample which has been obtained from said subject before or after initiating said treatment,
(b) determining the amounts of said at least three biomarkers in a second sample which has been obtained from said subject after said first sample, and
(c) comparing the amounts of said at least three biomarkers in said second sample to the amounts of said at least three biomarkers in said first sample, thereby monitoring the treatment of NASH.

2. The method of claim 1, wherein said subject is human.

3. The method of claims 1 and 2, wherein said sample is a blood, serum or plasma sample.

4. The method any one of claims 1 to 3, wherein said second sample has been obtained at least one month after said first sample.

5. The method according to any one of claims 1 to 4, wherein amounts of said at least three biomarkers in said second sample which differ from the amounts in said first sample are indicative for a change of the status of NASH in said subject.

6. The method of any one of claims 1 to 5, wherein the comparison of the amounts in step c) comprises i) the calculation of a first score based on the amounts of the at least three biomarkers in the first sample, ii) the calculation of a second score based on the amounts of the at least three biomarkers in the second sample, and iii) the comparison of the second score to the first score.

7. The method of any one of claims 1 to 6, wherein said treatment of NASH is gastric bypass surgery.

8. The method of any one of claims 1 to 7, wherein said treatment of NASH is a drug-based treatment, in particular wherein said drug-based treatment comprises the administration of at least one drug selected from statins, incretin analogues, metformin, rimonabant, thiazolidinediones, and orlistat.

9. The method of any one of claims 1 to 8, further comprising i) the determination of the amount of the cytokeratin 18 fragment (M30) in said first and said second sample, and ii) the comparison of the amount of said fragment in said second sample to the amount in said first sample,
in particular wherein the amount of the cytokeratin 18 fragment (M30) is determined by using a detection agent which specifically detects the cytokeratin 18 fragment (M30) such as an antibody, or antigen-binding fragment thereof.

10. The method of any one claims 1 to 9, wherein miR-193b is one of the at least three biomarkers.

11. The method of claim 10, wherein the biomarkers miR-193b and miR-365a are two of the at least three biomarkers.

12. The method of any one of claims 1 to 11, further comprising i) the determination of the amount of at least one further miRNA biomarker in said first and said second sample, wherein said at least one further miRNA biomarker is selected from the group consisting of miR-1260a, miR-30a, and miR-34a, and ii) the comparison of the amount of said at least one further miRNA biomarker in said second sample to the amount in said first sample.

13. A method for diagnosing non-alcoholic steatohepatitis (NASH) in a subject suspected to suffer from NASH, comprising
(a) determining the amounts of at least three biomarkers selected from the group consisting of miR-193b, miR-365a, miR-99a, miR-28, let-7c, miR-192, miR-215, and miR-122 in a sample from said subject, and
(b) comparing the amounts of said at least three biomarkers to a reference, thereby diagnosing NASH.

14. A method for assessing whether a test compound is capable of treating NASH, comprising:
(d) determining the amounts of at least three biomarkers selected from the group consisting of miR-193b, miR-365a, miR-99a, miR-28, let-7c, miR-192, miR-215, and miR-122 in a first sample which has been obtained from a subject suffering from NASH before or after initiating administration of said test compound,
(e) determining the amounts of said at least three biomarkers in a second sample which has been obtained from said subject after said first sample, and
(f) comparing the amounts of said at least three biomarkers in said second sample to the amount of said at least three biomarkers in said first sample, thereby assessing whether said test compound is capable of treating NASH.

15. *In vitro* use of at least three biomarkers selected from the group consisting of miR-193b, miR-365a, miR-99a, miR-28, let-7c, miR-192, miR-215, and miR-122 as biomarkers, or of at least three detection agents selected from the group consisting of a detection agent which specifically detects miR-193b, a detection agent which specifically detects miR-365a, a detection agent which specifically detects miR-99a, a detection agent which specifically detects miR-28, a detection agent which specifically detects let-7c, a detection agent which specifically detects miR-192, a detection agent which specifically detects miR-215, and a detection agent which specifically detects miR-122,
(i) in a first and second sample from a subject suffering from NASH, for monitoring a treatment of NASH in said subject, wherein said first sample has been obtained from said subject before or after initiating said treatment, and wherein said second sample has been obtained from said subject after said first sample, or
(ii) in a sample from a subject suspected to suffer from NASH for diagnosing whether said subject suffers from NASH, or
(iii)in a first and second sample from a subject suffering from NASH for assessing whether a test compound is capable of treating NASH, wherein said first sample has been obtained from said subject before or after initiating administration of said test compound, and wherein said second sample has been obtained after said first sample.
